# EUROPÄISCHE PATENTSCHRIFT

(11) **EP 1 336 885 B1**
(45) Veröffentlichungstag und Bekanntmachung des Hinweises auf die Patenterteilung: **17.12.2003**
(21) Anmeldenummer: 02003355.1
(22) Anmeldetag: 13.02.2002
(51) Int. Cl.: G02B 7/00, A61B 19/00

(54) **Mikrochirurgisches Mikroskopsystem**
Microsurgical microscope system
Système microscopique microchirurgical

(43) Veröffentlichungstag der Anmeldung: 20.08.2003
(73) Patentinhaber: Möller-Wedel GmbH, 22880 Wedel (DE)
(72) Erfinder: Schmidt, Martin, Dr., 23611 Bad Schwartau (DE)
(74) Vertreter: Glawe, Delfs, Moll & Partner

(56) Entgegenhaltungen:
- EP-A- 0 628 290
- WO-A-98/53244
- US-A- 5 061 018

## Beschreibung

Die Erfindung betrifft ein mikrochirurgisches Mikroskopsystem mit einem Stativ und einem daran angebrachten Mikroskop, bei dem das Stativ aufweist: ein Grundteil, ein erstes im wesentlichen vertikales Parallelogrammgestänge, von dem eine der im wesentlichen vertikalen Stangen am Grundteil um eine Schwenkachse schwenkbar gelagert ist, ein zweites im wesentlichen horizontales Parallelogrammgestänge mit zwei im wesentlichen horizontalen Stangen und zwei weiteren Stangen, dessen eine im wesentlichen horizontale Stange eine Verlängerung der oberen im wesentlichen horizontalen Stange des ersten Parallelogrammgestänges ist und an deren vom ersten Parallelogrammgestänge entfernt angeordneten ersten weiteren Stange eine Anbringungseinrichtung für das Mikroskop angebracht ist, und ein drittes im wesentlichen vertikales Parallelogrammgestänge, dessen erstes unteres Gelenk an der Schwenkachse der einen der im wesentlichen vertikalen Stangen des ersten Parallelogrammgestänges mit dem Grundteil und dessen zweites unteres Gelenk ebenfalls mit dem Grundteil verbunden ist und das über seine obere Stange mit dem zweiten Parallelogrammgestänge verbunden ist.

Für Operationen an feinen biologischen Strukturen, insbesondere für neurochirurgische Operationen an Gehirn und Rückenmark, aber auch im HNO-Bereich, werden Operationsmikroskope verwendet, die an leicht beweglichen Stativen nahe dem Operationsfeld angebracht sind.

Je nach Operationsart werden Operationsmikroskope und Stative unterschiedlicher Größe und Bauart eingesetzt. In der Neurochirurgie haben sich Systeme besonders bewährt, bei denen das Stativ Gewichte enthält, die nach Art einer Waage das Gewicht des Operationsmikroskops ausgleichen. Da aber das Mikroskop in den drei Raumrichtungen bewegt werden muß, muß das Stativ mindestens drei Bewegungsachsen aufweisen. Während eine Achse vertikal angeordnet sein kann und daher um diese Achse kein Gewichtsausgleich zu erfolgen hat, ist es erforderlich, die Balance um zwei Achsen durch Veränderung der Gewichte oder Verschieben von Gewichten herzustellen.

Beispiele solcher Stative können zahlreich der Patentliteratur entnommen werden. Insbesondere beschreibt die Schrift US 5,528,417 ein System, bei dem ein Ausgleich des Mikroskopgewichts durch ein Gegengewicht erfolgt, das je nach dem Mikroskopgewicht auf einer Kurve verschoben wird. Bei diesem System wird zusätzlich die Aufnahme des Mikroskops durch Schubstangen waagerecht gehalten, so daß das Mikroskop immer senkrecht unter der Aufnahme hängt.

Bei dem vorbekannten Mikroskopsystem sind das zweite und das dritte Parallelogrammgestänge über einen Hebel mit zwei Schenkeln verbunden, die einen Winkel von 90° mit sich einschließen. Dabei wird der untere Schenkel immer waagerecht, der obere Schenkel immer senkrecht gehalten. Dies ergibt zwar einen recht großen Schwenkbereich für das zweite Parallelogrammgestänge und damit das daran angebrachte Mikroskop. In der Normalstellung, wenn das zweite Parallelogramm ein Rechteck bildet, ist das zweite Parallelogramm aber waagerecht, so daß der Operateur mit dem Kopf daran stoßen kann. Aus diesem Grunde ist es beim Stand der Technik erforderlich, die im wesentlichen horizontalen Stangen erhöht anzubringen bzw. eine sich vom Operationsgebiet entfernende Krümmung aufweisen zu lassen.

Die Aufgabe der Erfindung besteht in der Schaffung eines Mikroskopsystems, das sehr bedienungsfreundlich ist, indem es mehr Platz für den Operateur freiläßt.

Die erfindungsgemäße Lösung besteht darin, daß das zweite untere Gelenk des dritten Parallelogrammgestänges höher angeordnet ist als das erste und daß die Verbindungslinie zwischen erstem und zweiten Gelenk mit der Horizontalen einen Winkel von ca. 30° bis 60° bildet und daß die obere Stange des dritten Parallelogrammgestänges die zweite weitere Stange des zweiten Parallelogrammgestänges bildet.

In diesem Fall steht die Stange des zweiten Parallelogrammgestänges, die sich in der Nähe des ersten und dritten Parallelogrammgestänges befindet, nicht senkrecht, sondern unter einem Winkel von ungefähr 30° bis 60°, vorzugsweise unter 45°, so daß in dieser Normalstellung das zweite Parallelogrammgestänge schräg nach oben gerichtet ist und der Operateur darunter bequem Platz findet.

Je nach der Operation werden am Operationsmikroskop unterschiedliche Zusatzteile benötigt, die das Gewicht des Operationsmikroskops verändern. Daher muß nach Umrüstung des Mikroskops das System neu balanciert werden.

Der regelmäßig erforderliche Gewichtsausgleich bringt jedoch Nachteile. Bei Ausgleich der Gewichtsverschiebung ist der Ausgleichsbereich begrenzt und muß ggf. durch Anbringung zusätzlicher Gewichte am Stativ erweitert werden. Bei automatischer Balancierung wird ein hoher elektromechanischer Aufwand erforderlich, der die Kosten für ein System erheblich in die Höhe treibt. Wird vergessen, die Balancierung durchzuführen, ist die Folge eine Unannehmlichkeit für den Chirurgen bis hin zu einer Gefährdung des Patienten durch unkontrollierte Bewegungen des Mikroskops.

Aus den genannten Gründen werden die Mikroskope mit allen möglichen Zubehörteilen ausgerüstet, auch wenn diese für eine Operation nicht benötigt werden. Dadurch vermeidet man deine Balancierung vor der Operation um die damit verbundene Gefahr des Ungleichgewichts. Der Nachteil ist jedoch, daß das Mikroskop durch die vielen Zubehörteile groß und unhandlich wird und den Sichtkontakt auf das Operationsfeld über Gebühr behindert.

Die vorstehenden Nachteile können vermieden werden, wenn erfindungsgemäß vorgesehen ist, daß mit der ersten weiteren Stange des zweiten Parallelogrammgestänges eine Aufnahmeeinrichtung für Zubehörteile des Mikroskops und/oder Zusatzgewichte verbunden ist.

Die Erfindung nutzt dabei die Erkenntnis, daß wegen des besonderen Aufbaus des Mikroskopsystems sich die Balancierung nicht ändert, wenn sich das auf das zweite Parallelogrammgestänge wirkende Gewicht nicht ändert. Dieses Gewicht ändert sich aber nicht, wenn Teile vom Mikroskop, das mit der ersten weiteren Stange des zweiten Parallelogrammgestänges verbunden ist, entfernt werden und in oder an der Aufnahmeeinrichtung für die Zubehörteile angebracht werden, die ebenfalls mit der ersten weiteren Stange des zweiten Parallelogrammgestänges verbunden ist. Daß sich dabei die Entfernung dieser Gegenstände vom Grundteil oder irgendwelchen Gelenken der Parallelogrammgestänge ändert, was unterschiedliche Drehmomente hervorrufen würde, spielt dabei erstaunlicherweise keine Rolle. Dies beruht auf der Tatsache, daß sich auch bei Verschwenken der Parallelogrammgestänge die Orientierung der ersten weiteren Stange des zweiten Parallelogrammgestänges samt daran angebrachtem Mikroskop und Zubehörteilen nicht ändert.

Zubehörteile, die im Moment nicht benötigt werden, behindern daher nicht die Arbeit mit dem Mikroskop, da sie vom Mikroskop abgenommen werden können und in oder an der Aufnahmeeinrichtung für Zubehörteile angebracht werden können, wo sie die Arbeit mit dem Mikroskop nicht mehr stören. Die entsprechenden Teile brauchen nicht durch den Operationsraum und erst recht nicht in andere Räume verbracht werden, wobei die Gefahr bestehen würde, daß sie beschädigt, verlegt oder kontaminiert werden. Eine neue Balancierung nach Auswechseln eines Zubehörteiles ist nicht erforderlich. Die Balancierung kann vielmehr für ein vorgegebenes Mikroskop und einen vorgegebenen Satz von Zubehörteilen einmalig vorgenommen werden. Irgendwelche automatischen Balancierungseinrichtungen sind nicht mehr erforderlich. Das Stativ kann dadurch insgesamt leichter, kleiner und kostengünstiger gebaut werden.

Die Zubehörteile für das Mikroskop nehmen unter Umständen verhältnismäßig viel Raum ein, so daß es Schwierigkeiten bringt, sie am Ende des zweiten Parallelogrammgestänges in der Aufnahmeeinrichtung unterzubringen. In diesem Falle kann vorgesehen sein, daß am Grundteil eine Aufnahmeeinrichtung für Zubehörteile des Mikroskops und Zusatzgewichte vorgesehen ist. Für jedes Zubehörteil ist in diesem Falle ein gleich schweres Zusatzgewicht vorgesehen, das, wenn es z.B. aus massivem Metall ist, selbstverständlich ein wesentlich kleineres Volumen einnimmt als das Zubehörteil. Wird das verhältnismäßig großvolumige Zubehörteil nicht benötigt, so kann es in der Aufnahmeeinrichtung am Grundteil untergebracht werden, wo genügend Platz vorhanden ist. Statt dessen wird dann das wesentlich kleinere Zusatzgewicht in die Aufnahmeeinrichtung am äußeren Ende des zweiten Parallelogrammgestänges in der Nähe des Mikroskops angebracht.

Zweckmäßigerweise sind an Gelenken und/oder der Schwenkachse elektrisch oder pneumatisch lösbare Bremsen angeordnet. Diese Bremsen werden dann nur gelöst, wenn das Stativ verstellt werden soll, d.h., das Mikroskop in eine andere Lage gebracht werden soll.

Das Mikroskopsystem kann, nachdem alle erforderlichen Zubehörteile bzw. die entsprechenden Zusatzgewichte in der Aufnahmeeinrichtung am zweiten Parallelogrammgestänge bzw. dem Mikroskop angebracht sind, ausbalanciert werden. Dies geschieht zweckmäßigerweise durch an den Parallelogrammgestängen, insbesondere dem ersten Parallelogrammgestänge anbringbare Gewichte. Diese Balancierung kann einmalig von einem Techniker oder einer Operationsschwester für ein gegebenes System vorgenommen werden und kann so lange beibehalten werden, wie nicht die Zusatzgeräte für das Mikroskop durch andere ersetzt werden.

Eine besonders zweckmäßige Bauart zeichnet sich dadurch aus, daß die Stangen der Parallelogrammgestänge Rohre sind, die in Muffen gehalten werden, die Teile der Gelenke und/oder der Schwenkachse bilden. Dabei können die Gelenkachsen und/oder die Schwenkachse in den Muffen außerhalb der Längsachse der Rohre angeordnet sein. Um mehr Platz für den Operateur zu schaffen, kann mindestens eine Stange der Parallelogrammgestänge in vom Operationsfeld wegweisende Richtung gekrümmt sein.

Der Grundteil wird zweckmäßigerweise auf dem Fußboden stehen und kann in an sich bekannter Weise mit arretierbaren Rollen versehen sein. Er könnte aber auch z.B. an der Wand oder an der Decke des Operationssaals angebracht werden. Zweckmäßigerweise weist der Grundteil dabei einen Fußteil und eine um eine vertikale Achse schwenkbare Säule auf, an der dann die weiteren Teile des Stativs angebracht sind.

Das wesentliche an der Erfindung ist es aber, daß sich bei Verschwenken des Stativs der Winkel, unter dem das Mikroskop und die Aufnahmeeinrichtung für die Zubehörteile orientiert sind, nicht ändert. Ändert sich dann das Gesamtgewicht von Mikroskop und Zubehörteilen, die am Mikroskop oder an bzw. in der Aufnahmevorrichtung angeordnet sind, nicht, so bleibt die Balancierung erhalten.

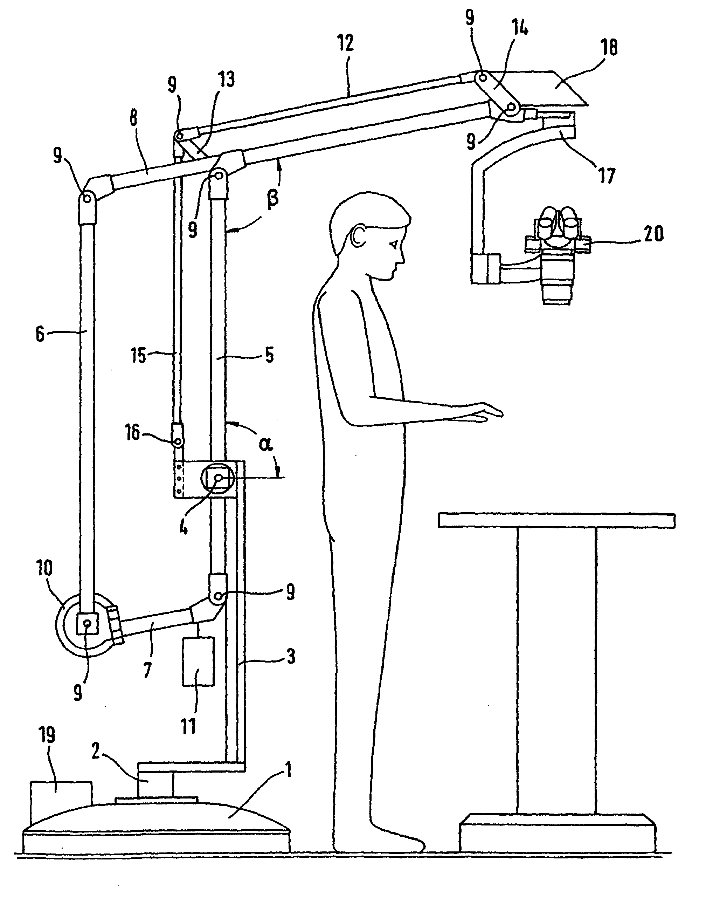

Die Erfindung wird im folgenden anhand einer vorteilhaften Ausführungsform unter Bezugnahme auf die beigefügte Zeichnung beschrieben, die das erfindungsgemäße Mikroskopsystem schematisch von der Seite zeigt.

An einem Fußteil 1 ist über ein Gelenk 2 eine um eine vertikale Achse schwenkbare Säule 3 angeordnet. An dieser Säule 3 ist um eine horizontale Schwenkachse 4 ein erstes Parallelogrammgestänge schwenkbar angebracht. Dieses weist vertikale Stangen 5, 6 sowie im wesentlichen horizontale Stangen 7, 8 auf, die jeweils durch Gelenke 9 verbunden sind. An dem unteren linken Gelenk 9 sind Ausgleichgewichte 10 angebracht, mit denen das Stativ ausbalanciert werden kann. Man kann auch, statt nur an einer Stelle solche Gewichte 10 vorzusehen, solche Gewichte auch an anderer Stelle, z.B. Gewichte 11 an der unteren Stange 7 vorsehen, wie dies in der Figur gezeigt ist. Auch könnten an der Stange 6 oder 7 verschiebbare Gewichte angeordnet sein.

Die obere Stange 8 des ersten Parallelogrammgestänges 5 bis 9 ist nach rechts verlängert und bildet hier den Teil eines zweiten Parallelogrammgestänges, das außer der genannten Stange 8 eine im wesentlichen horizontale Stange 12 und zwei weitere Stangen 13, 14 aufweist. Die Stangen 8, 12, 13, 14 sind ebenfalls durch Gelenke 9 zu einem Parallelogrammgestänge verbunden. Die Stange 13 bildet dabei einen Teil eines weiteren Parallelogrammgestänges, das zusätzlich zur Stange 13 einen Teil der Stange 5 des ersten Parallelogrammgestänges bis zur Schwenkachse 4, eine weitere im wesentlichen vertikale Stange 15, die bei 16 am Grundteil 1, 2, 3 gelagert ist, und die Verbindung zwischen Schwenkachse 4 und Gelenk 16 bildet. Die Verbindungslinie zwischen Schwenkachse 4 und Gelenk 16 steht dabei unter dem genannten Winkel von ungefähr 30° bis 60°, hier insbesondere 45° zur Horizontalen, wodurch die entsprechende Orientierung der Stange 13 festgelegt wird.

Die Anbringungsvorrichtung 17 für das Mikroskop ist mit der weiteren Stange 14 verbunden, die auch bei Verschwenken der Parallelogrammgestänge parallel zur Stange 13 bleibt und damit ihre Orientierung nicht ändert. Mit dieser ersten weiteren Stange 14 des zweiten Parallelogrammgestänges ist auch eine Aufnahmevorrichtung 18 für Zubehörteile des Mikroskops verbunden. In dieser Aufnahmevorrichtung 18 können nicht nur in der Figur nicht gezeigte Zubehörteile des Mikroskops untergebracht werden, sondern auch Zusatzgewichte, deren Masse der Masse von Zubehörteilen entspricht, die bei Nichtgebrauch in einer Aufnahmevorrichtung 19 am Fuß 1 des Mikroskops untergebracht werden können. Das Mikroskop selbst ist bei 20 gezeigt.

Das Drehmoment, das durch das Mikroskop 28, die Aufnahmeeinrichtung 18 und die Gewichte der Stangen 8, 12, 13, 14 und die übrigen Teile des zweiten horizontalen Parallelogrammgestänges ausgeübt wird, kann, wenn die Stange 15 festgehalten wird, durch das Gegengewicht 10 perfekt ausgeglichen werden. Erlaubt man die Rotation der Stange 5 um die Schwenkachse 4 ist es möglich, daß das Stativ in eine Extremstellung fällt. Ist der Schwerpunkt des gesamten Systems über der Achse 4, streckt sich das System vollständig, liegt er unter der Schwenkachse 4, bewegt sich die Stange 5 solange, bis der Winkel α 90° erreicht. Durch das weitere Gewicht 11 kann das Stativ nun so ausgeglichen werden, daß der Schwerpunkt in der Schwenkachse 4 liegt und damit das Mikroskopgewicht unabhängig von den Winkeln α und β in jeder Position ausgeglichen ist.

Die Gewichte 10 und 11 können an verschiedenen Stellen positioniert werden. So kann das Gewicht 10 an jedem Punkt der Stange 6 befestigt werden. Auch kann das Gewicht 10 auf der Stange 7 oder deren Verlängerung befestigt werden, wobei zum Ausgleich der Momente dann das Gewicht 10 vergrößert oder verkleinert werden muß. Auch das Gewicht 11 kann auf der Stange 7 verschoben werden oder an der Stange 5 unterhalb der Schwenkachse 4 bzw. an einer Verlängerung der Stange 5 nach unten angebracht werden. Im letzteren Fall ist ebenfalls eine Variation des Gewichts 11 entsprechend dem Abstand von der Schwenkachse 4 erforderlich. Auch können die Gewichte 10, 11 teilweise oder vollständig in einem Gewicht zusammengefaßt werden. Auch können die Gewichte oder Teile davon innerhalb der Stangen 5, 6 oder 7 angebracht werden. Dadurch ist es möglich, die Breite des Stativs so gering wie möglich zu halten und die Auslenkung von Gegengewichten zu minimieren, wodurch die Zugänglichkeit für die Operationsschwester zum Operationsfeld optimiert wird.

An mindestens zwei Gelenken 9 des Stativs werden Bremsen angebracht, die durch elektrische Betätigung geöffnet oder geschlossen werden können. Soll das Mikroskop 20 im Raum bewegt werden, werden per Knopfdruck an einem Handgriff des Operationsmikroskops alle Bremsen gelöst, wodurch das Mikroskop mit minimalem Kraftaufwand in jede Position innerhalb des Bewegungsbereichs des Mikroskops 20 gebracht werden kann. Vorzugsweise sind diese Bremsen im Gelenk 9 bei dem Ausgleichsgewicht 10 und in der Schwenkachse 4 angeordnet, so daß das Gewicht der Bremsen entweder keinen Einfluß hat oder zum Gewichtsausgleich beiträgt. Auch der Transformator für die Stromversorgung ist vorzugsweise beim Gelenk 9 beim Gewicht 10 angeordnet.

Die Gewichte 10 und 11 werden so gewählt, daß bei einem maximalen Gewicht des Operationsmikroskops das Stativ vollständig ausgeglichen ist. Dann werden die Gewichte 10 und 11 fixiert.

An der Mikroskopanbringungseinrichtung 17 befindet sich eine Aufnahmeeinrichtung 18, an bzw. in der die Zubehörteile des Mikroskops angebracht werden können. Diese Aufnahme kann entweder ein Kasten sein oder eine Halterung mit Ringschwalben ähnlich den Befestigungsmöglichkeiten am Operationsmikroskop oder eine Kombination aus beidem. Wechselt beispielsweise der Arzt von einer Schädeloperation, bei der der Assistent seitlich vom Operateur steht zu einer Wirbelsäulenoperation, bei der der Assistent gegenüber dem Operateur auf der anderen Seite des Operationstisches sitzt, braucht die OP-Schwester zur Vorbereitung der Operation lediglich den Mitbeoachtertubus vom Operationsmikroskop zu entfernen und in der Aufnahmeeinrichtung 18 an der Mikroskopanbringungseinrichtung 17 zu deponieren. Umgekehrt wird sie der Aufnahmeeinrichtung einen zweiten Einblick entnehmen und am Operationsmikroskop 20 gegenüber dem Operateur anbringen. Auf diese Weise bleibt das Stativ vollständig gewichtsausgeglichen, ohne daß Gewichte verschoben oder Bedienungsknöpfe betätigt werden müssen. Auch entfällt die Notwendigkeit, daß die OP-Schwester mit einem teuren optischen Element durch den OP-Saal oder zu einem Aufbewahrungsort gehen muß und dabei Gefahr läuft, das optische Element zu beschädigen.

Soll von Zeit zu Zeit die Gesamtkonfiguration des Mikroskops geändert werden, z.B. durch Anschluß einer leichteren Videokamera, muß das Stativ nur in diesen Fällen neu ausgeglichen werden. Dieser Ausgleich sollte vorzugsweise von einem Servicetechniker oder einem Krankenhausmechaniker vorgenommen werden. Dabei werden vorzugsweise Gewichtselemente entweder bei den Ausgleichsgewichten 10 und 11 entnommen bzw. eingesetzt oder andere Gewichtselemente im Bereich der Mikroskopanbringungseinrichtung 17 oder der Aufnahmeeinrichtung 18 hinzugefügt bzw. entfernt. Dazu wird mit dem Operationssystem ein Gewichtssatz geliefert. Außerdem ist die Bremsensteuerung so ausgelegt, daß die Bremsen in der Schwenkachse 4 und dem Gelenk 16 getrennt geöffnet werden können. Für einen Techniker ist es dann sehr einfach, einen perfekten Gewichtsausgleich mit dem neuen Mikroskopgewicht wieder herzustellen.

Zur Feineinstellung bei der Installation oder nach einer späteren Änderung des Traggewichtes können kleinere Gewichte an den Stangen 5, 6 oder 7 angebracht werden, die verschoben und dann fixiert werden. Auch ist es möglich, das Gewicht 10 oder 11 oder beide geringfügig verschiebbar zu machen, um dadurch die Feineinstellung durchzuführen.

Die Stangen 5, 6, 7, 8, 12, 13, 14, 15 sind vorzugsweise als Rohre ausgebildet, wobei verschiedene Querschnittsformen möglich sind. Die Rohre können durch Muffen miteinander verbunden werden, in denen sich die Gelenke für die Bewegung befinden. In diesem Fall ist es vorteilhaft, wenn sich die Gelenkachsen außerhalb der Rohre befinden. Ist nämlich das Rohr 8 oberhalb der Gelenke angeordnet, gewinnt man an Höhe für den Chirurgen, wenn er unterhalb der Stangen des zweiten Parallelogrammgestänges operiert. Um noch größere Höhe zu gewinnen, ist es auch möglich, die Stange 8 mit einer nach oben gerichteten Biegung zu versehen, in dem Bereich, in dem sich der Chirurg bei der Operation aufhalten kann.

Die Aufnahmeeinrichtung 18 muß - wie schon beschrieben - von der Größe her geeignet sein, die Zubehörteile des Operationsmikroskops 20 aufzunehmen. Sollen viele Teile von einer Operation zur nächsten gewechselt werden, kann man auch daran denken, diese Teile anderswo unterzubringen, z.B. in einem

Fach 19 am Stativfuß 1, und statt dessen unterschiedliche Gewichte diesem Fach 19 zu entnehmen und in der Aufnahmeeinrichtung 18 anzubringen. Da diese Gewichte ein wesentlich kleineres Volumen haben können, kann die Aufnahmeeinrichtung 18 erheblich kleiner ausfallen.

Wenn bei den Parallelogrammgestängen von Stangen die Rede ist, so sind damit die Parallelogrammseiten gemeint. Eine dieser Parallelogrammseiten zwischen Schwenkachse 4 und Gelenk 16 ist natürlich keine solche Stange bzw. braucht keine solche Stange zu sein. Dies ist auch für andere sog. Stangen der Fall, insbesondere die Stange 14. Statt eine Stange 14 vorzusehen, könnten die Stangen 8 und 12 mit Gelenken 9 direkt an der Aufnahmeeinrichtung 18 verbunden sein.

## Patentansprüche

1. Mikrochirurgisches Mikroskopsystem mit einem Stativ und einem daran angebrachten Mikroskop (20), bei dem das Stativ aufweist:
- ein Grundteil (1, 2, 3),
- ein erstes im wesentlichen vertikales Parallelogrammgestänge (5, 6, 7, 8), von dem eine (5) der im wesentlichen vertikalen Stangen (5, 6, 7, 8) am Grundteil (1 ,2 ,3) um eine Schwenkachse (4) schwenkbar gelagert ist,
- ein zweites im wesentlichen horizontales Parallelogrammgestänge (8, 12, 13 ,14) mit zwei im wesentlichen horizontalen Stangen (8, 12) und zwei weiteren Stangen (13, 14), dessen eine im wesentlichen horizontale Stange (8) eine Verlängerung der oberen im wesentlichen horizontalen Stange (8) des ersten Parallelogrammgestänges (5, 6, 7, 8) ist und an deren vom ersten Parallelogrammgestänge (5, 6, 7, 8) entfernt angeordneten ersten weiteren Stange (14) eine Anbringungseinrichtung (17) für das Mikroskop (20) angebracht ist, und
- ein drittes im wesentlichen vertikales Parallelogrammgestänge (5, 13, 15), dessen erstes unteres Gelenk an der Schwenkachse (4) der einen der im wesentlichen vertikalen Stangen (5) des ersten Parallelogrammgestänges (5, 6, 7, 8) mit dem Grundteil (1, 2, 3) und dessen zweites unteres Gelenk (16) ebenfalls mit dem Grundteil (1, 2, 3) verbunden ist und das über seine obere Stange (13) mit dem zweiten Parallelogrammgestänge (8, 12, 13, 14) verbunden ist,
**dadurch gekennzeichnet, daß** das zweite untere Gelenk (16) des dritten Parallelogrammgestänges (5, 13, 15) höher angeordnet ist als das erste (4) und daß die Verbindungslinie zwischen erstem und zweiten Gelenk (4, 16) mit der Horizontalen einen Winkel von ca. 30° bis 60° bildet und daß die obere Stange (13) des dritten Parallelogrammgestänges (5, 15) die zweite weitere Stange des zweiten Parallelogrammgestänges (8, 12, 13, 14) bildet.

2. Mikroskopsystem nach Anspruch 1, **dadurch gekennzeichnet, daß** die Verbindungslinie zwischen erstem und zweiten Gelenk (4, 16) mit der Horizontalen einen Winkel von ca. 45° bildet.

3. Mikroskopsystem nach Anspruch 1 oder 2, **dadurch gekennzeichnet, daß** mit der ersten weiteren Stange (14) des zweiten Parallelogrammgestänges (8, 12, 13, 14) eine Aufnahmeeinrichtung (18) für Zubehörteile des Mikroskops (20) und/oder Zusatzgewichte verbunden ist.

4. Mikroskopsystem nach einem der Ansprüche 1 bis 3, **dadurch gekennzeichnet, daß** am Grundteil (1, 2, 3) eine Aufnahmeeinrichtung (19) für Zubehörteile des Mikroskops (20) und Zusatzgewichte vorgesehen ist.

5. Mikroskopsystem nach einem der Ansprüche 1 bis 4, **dadurch gekennzeichnet, daß** an Gelenken (9, 16) und/oder der Schwenkachse (4) elektrisch oder pneumatisch lösbare Bremsen angeordnet sind.

6. Mikroskopsystem nach einem der Ansprüche 1 bis 5, **dadurch gekennzeichnet, daß** es an den Parallelogrammgestängen, insbesondere dem ersten Parallelogrammgestänge (5, 6, 7, 8) anbringbare Gewichte (10, 11) aufweist.

7. Mikroskopsystem nach einem der Ansprüche 1 bis 6, **dadurch gekennzeichnet, daß** die Stangen (5, 6, 7, 8, 12, 13, 14) Rohre sind, die in Muffen gehalten werden, die Teile der Gelenke (9, 16) und/oder der schwenkachse (4) bilden.

8. Mikroskopsystem nach Anspruch 7, **dadurch gekennzeichnet, daß** die Gelenkachsen (9, 16) und/oder die Schwenkachse (4) in den Muffen außerhalb der Längsachse der Rohre (5, 6, 7, 8, 12, 13, 14) angeordnet sind.

9. Mikroskopsystem nach einem der Ansprüche 1 bis 8, **dadurch gekennzeichnet, daß** mindestens eine Stange (8) der Parallelogrammgestänge in vom operationsfeld wegweisende Richtung gekrümmt ist.

10. Mikroskopsystem nach einem der Ansprüche 1 bis 9, **dadurch gekennzeichnet, daß** der Grundteil (1, 2, 3) einen Fußteil (1) und eine um eine vertikale Achse (2) schwenkbare Säule (3) aufweist.

## Claims

1. Microsurgical microscope system with a stand and with a microscope (20) arranged thereon, in which system the stand has:
- a base part (1, 2, 3),
- a first substantially vertical parallelogram linkage (5, 6, 7, 8), of which one bar (5) of the substantially vertical bars (5, 6, 7, 8) is mounted on the base part (1, 2, 3) so as to pivot about a pivot axle (4)
- a second substantially horizontal parallelogram linkage (8, 12, 13, 14) with two substantially horizontal bars (8, 12) and two further bars (13, 14), of which one substantially horizontal bar (8) is a continuation of the upper substantially horizontal bar (8) of the first parallelogram linkage (5, 6, 7, 8), and a mounting device (17) for the microscope (20) is arranged on the first further bar (14) arranged at a distance from the first parallelogram linkage (5, 6, 7, 8), and
- a third substantially vertical parallelogram linkage (5, 13, 15) whose first lower hinge on the pivot axle (4) of one of the substantially vertical bars (5) of the first parallelogram linkage (5, 6, 7, 8) is connected to the base part (1, 2, 3), and whose second lower hinge (16) is likewise connected to the base part (1, 2, 3) and is connected via its upper bar (13) to the second parallelogram linkage (8, 12, 13, 14),
**characterized in that** the second lower hinge (16) of the third parallelogram linkage (5, 13, 15) is arranged higher than the first (4), and **in that** the connection line between first and second hinges (4, 16) forms with the horizontal an angle of ca. 30° to 60°, and **in that** the upper bar (13) of the third parallelogram linkage (5, 15) forms the second further bar of the second parallelogram linkage (8, 12, 13, 14).

2. Microscope system according to Claim 1, **characterized in that** the connection line between first and second hinges (4, 16) forms with the horizontal an angle of ca. 45°.

3. Microscope system according to Claim 1 or 2, **characterized in that** a receiving device (18) for accessory parts of the microscope (20) and/or additional weights is connected to the first further bar (14) of the second parallelogram linkage (8, 12, 13, 14).

4. Microscope system according to one of Claims 1 to 3, **characterized in that** a receiving device (19) for accessory parts of the microscope (20) and additional weights is provided on the base part (1, 2, 3).

5. Microscope system according to one of Claims 1 to 4, **characterized in that** electrically or pneumatically releasable brakes are arranged on hinges (9, 16) and/or the pivot axle (4).

6. Microscope system according to one of Claims 1 to 5, **characterized in that** it has weights (10, 11) which can be arranged on the parallelogram linkages, in particular on the first parallelogram linkage (5, 6, 7, 8).

7. Microscope system according to one of Claims 1 to 6, **characterized in that** the bars (5, 6, 7, 8, 12, 13, 14) are pipes which are held in bushings which form parts of the hinges (9, 16) and/or of the pivot axle (4).

8. Microscope system according to Claim 7, **characterized in that** the hinge axles (9, 16) and/or the pivot axle (4) are arranged in the bushings outside the longitudinal axis of the pipes (5, 6, 7, 8, 12, 13, 14).

9. Microscope system according to one of Claims 1 to 8, **characterized in that** at least one bar (8) of the parallelogram linkage is curved in the direction away from the operating site.

10. Microscope system according to one of Claims 1 to 9, **characterized in that** the base part (1, 2, 3) has a foot part (1) and a column (3) which is pivotable about a vertical shaft (2).

## Revendications

1. Système de microscope microchirurgical comportant un trépied sur lequel un microscope (20) est monté, dans lequel le trépied présente :
- une partie de base (1, 2, 3),
- une première tringlerie en parallélogramme essentiellement verticale (5, 6, 7, 8) dont l'une (5) des barres essentiellement verticales (5, 6, 7, 8) est montée sur la partie de base (1, 2, 3), de manière à pouvoir pivoter autour d'un axe de pivotement (4),
- une deuxième tringlerie en parallélogramme essentiellement horizontale (8, 12, 13, 14) comprenant deux barres essentiellement horizontales (8, 12) et deux autres barres (13, 14), dont une barre essentiellement horizontale (8) est un prolongement de la barre supérieure essentiellement horizontale (8) de la première tringlerie en parallélogramme (5, 6, 7, 8) et sur la première autre barre (14) de laquelle, disposée de manière éloignée de la première tringlerie en parallélogramme (5, 6, 7, 8), un dispositif de montage (17) pour le microscope (20) est monté, et
- une troisième tringlerie en parallélogramme essentiellement verticale (5, 13, 15) dont la première articulation inférieure sur l'axe de pivotement (4) de l'une des barres essentiellement verticale (5) de la première tringlerie en parallélogramme (5, 6, 7, 8) est reliée avec la partie de base (1, 2, 3) et dont la deuxième articulation inférieure (16) est également reliée avec la partie de base (1, 2, 3) et qui est reliée par sa barre supérieure ( 13) avec la deuxième tringlerie en parallélogramme (8, 12, 13, 14),
**caractérisé en ce que** la deuxième articulation inférieure (16) de la troisième tringlerie en parallélogramme (5, 13, 15) est disposée plus haut que la première (4) et que la ligne de liaison entre la première et la deuxième articulation (4, 16) forme un angle d'environ 30° à 60° avec l'horizontale et que la barre supérieure (13) de la troisième tringlerie en parallélogramme (5, 15) forme la deuxième autre barre de la deuxième tringlerie en parallélogramme (8, 12, 13, 14).

2. Système de microscope selon la revendication 1, **caractérisé en ce que** la ligne de liaison entre la première et la deuxième articulation (4, 16) forme un angle d'environ 45° avec l'horizontale.

3. Système de microscope selon la revendication 1 ou 2, **caractérisé en ce que**, avec la première autre barre (14) de la deuxième tringlerie en parallélogramme (8, 12, 13, 14) est relié un dispositif de réception (18) pour des pièces accessoires du microscope (20) et/ou des poids complémentaires.

4. Système de microscope selon l'une quelconque de revendications 1 à 3, **caractérisé en ce que** sur la partie de base (1, 2, 3), un dispositif de réception (19) pour des pièces accessoires du microscope (20) et des poids complémentaires est prévu.

5. Système de microscope selon l'une quelconque de revendications 1 à 4, **caractérisé en ce que** les articulations (9, 16) et/ou sur l'axe de pivotement (4), des freins qu'il est possible de relâcher électriquement ou pneumatiquement sont disposés.

6. Système de microscope selon l'une quelconque de revendications 1 à 5, **caractérisé en ce qu'**il présente des poids (10, 11) pouvant être montés sur les tringleries en parallélogramme, en particulier à la première tringlerie en parallélogramme (5, 6, 7, 8).

7. Système de microscope selon l'une quelconque de revendications 1 à 6, **caractérisé en ce que** les barres (5, 6, 7, 8, 12, 13, 14) sont des tubes qui sont maintenus dans des manchons, qui forment des parties des articulations (9, 16) et/ou de l'axe de pivotement (4).

8. Système de microscope selon la revendication 7, **caractérisé en ce que** les axes d'articulation (9, 16) et/ou l'axe de pivotement (4) sont disposés dans les manchons, à l'extérieur de l'axe longitudinal des tubes (5, 6, 7, 8, 12, 13, 14).

9. Système de microscope selon l'une quelconque de revendications 1 à 8, **caractérisé en ce qu'**au moins une barre (8) de la tringlerie en parallélogramme est recourbée dans une direction s'écartant du champ d'opération.

10. Système de microscope selon l'une quelconque de revendications 1 à 9, **caractérisé en ce que** la partie de base (1, 2, 3) présente une pièce de pied (1) et une colonne (3) pouvant pivoter autour d'un axe vertical (2).
